# EUROPEAN PATENT SPECIFICATION

(11) **EP 0 402 925 B1**
(45) Date of publication and mention of the grant of the patent: **24.09.1997**
(21) Application number: 90111246.6
(22) Date of filing: 14.06.1990
(51) Int. Cl.: C07D 493/22, A61K 35/78

(54) **A method of isolating ginkgolides from the leaves of the ginkgo tree and purifying them**
Verfahren zur Gewinnung von Ginkgoliden aus Ginkgoblättern und ihre Reinigung
Méthode pour isoler des ginkgolides à partir de feuilles de ginkgos et leur purification

(30) Priority: 16.06.1989 KR 898340
(43) Date of publication of application: 19.12.1990
(73) Proprietor: SUNKYONG INDUSTRIES LTD., Jangan-gu, Suwon, Kyonggi-do 440-745 (KR)
(72) Inventor: Kwak, Wie Jong, Seoul (KR); Park, Hwa Kun, Kyonggi-do (KR); Oh, Key Bong, Kyongsangnam-do (KR)
(74) Representative: Weber, Dieter, Dr.

(56) References cited:
- EP-A- 0 086 315
- DE-A- 2 117 429
- JUSTUS LIEBIGS ANNALEN DER CHEMIE. no. 6, 1987, WEINHEIM DE pages 521 - 526; K. WEINGES ET AL.: 'Chemie der Ginkgolide, II. Isolierung und Strukturaufklärung eines neuen Ginkgolids'
- LIEBIGS ANNALEN DER CHEMIE. vol. 759, 1972, WEINHEIM DE pages 158 - 172; K. WEINGES ET AL.: 'NMR und massensspektrometrischer Vergleich des Bibobalids C15H18O8 mit den Ginkgoliden C20H24O9-11'
- TETRAHEDRON LETTERS. no. 4, 1967, OXFORD GB pages 299 - 302; M. MARUYAMA ET AL. : 'The ginkgolides. I. Isolation and characterization of the various groups'
- JOURNAL OF THE CHEMICAL SOCIETY, SECTION C: ORGANIC CHEMISTRY. no. 21, 1967, LETCHWORTH GB pages 2201 - 2206; K. OKABE ET AL.: 'Ginkgolides'
- Liebigs Ann.Chem. 1772(1986)
- Gingkolides-Chemistry,Biology,Pharmacology and Clinical Perspectives,(P.BRAQUET,Ed.)1988,J.R.PROUS Science Pub., p.37-42

## Description

The present invention relates to a method of isolating the effective physio-active substances ginkgolides from the leaves of the Ginkgo tree and purifying them, on a large scale.

It is known that there are many effective physio-active substances contained in the Ginkgo tree: the phenolic compounds ginkgel, bilobal, ginkgolic acid, etc. in the fruit; cyanogenetic glucosides, amino acids etc. in the seeds; aromatic compounds, particularly phenolic compounds, flavonoid glycosides and simple flavonoids - kaempferol, guercetin, isochamnetin, lutedin, etc. - terpenoid compounds ginkgolides and bilobalid, etc. in the leaves.

Particularly, as for the terpenoid compounds among the aforesaid compounds present in the leaves of the Ginkgo tree, the lactone compounds were first identified by S. Furukawa in 1929 (Sci. Papers Inst. Phys. Chem. Res. Tokyo [7. 27, 1932]); the ginkgolides were first isolated from the roots of the Ginkgo tree by K. Nakanishi in 1961 (Pure Appl. Chem. 1. 89 (1961), Tetrahedron Letter, 4. 299 (1961), and their structures were found out and named Ginkgolide A, B, C and M; in 1961, K. Okabe et al ascertained the presence of the ginkgolides also in the leaves of the Ginkgo tree (J. Chem. Soc. [5] 2201 [1961]).

After then, another ginkgolide, Ginkgolide J, was identified by K. Weing in 1981 (Lieb. Ann. Chem. 521 - 526 [1981]) and its chemical structural formula is as follows: 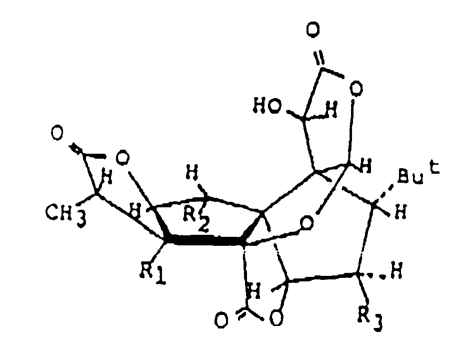 in the above structural formula

| **Kind of ginkgolides** | **R**_{**1**} | **R**_{**2**} | **R**_{**3**} |
|---|---|---|---|
| Ginkgolide A | -OH | -H | -H |
| Ginkgolide B | -OH | -OH | -H |
| Ginkgolide C | -OH | -OH | -OH |
| Ginkgolide M | -OH | -OH | -OH |
| Ginkgolide J | -OH | -H | -OH |

Recently, it has been found that, by their competitive reaction with platelet activating factor (PAF, PAF-acether, AGEPC), such ginkgolides have effects on many PAF-acether-induced diseases such as asthma, bronchitis, dementia senilis, alergy, cardiac disorders, shock related to organ transplantation, rhematic di-seases, etc. and other broad range of circulatory system diseases (P. Braguet, Drug of the Future, 12, 643, 1981).

According to the EP-A-0 086 315 an aqueous ketone extract of the leaves of the Ginkgo tree is combined with ammonium sulfate and an aliphatic ketone insoluble in water, the separated ketone phase is concentrated and then diluted by an aqueous alkanol, acidified and treated by means of a vinyl pyrrolidone polymer. A similar method is disclosed by the DE-A-2 117 429 according to which instead of the treatment with vinyl pyrrolidone polymer a treatment with a lead hydroxide suspension is carried out.

J. Chem. Soc. (C), page 2204 (1967) shows a method of isolating ginkgolides by combining the aqueous extract of the leaves of the Ginkgo tree with charcoal to adsorb the ginkgolides, the charcoal being extracted with acetone which is concentrated to obtain crystals. According to Liebigs Ann. Chem. 758, page 167 (1972) the aqueous extract of the Ginkgo tree leaves is extracted with acetic ester which is concentrated. The concentrate is dissolved in ethanol and the obtained solution is then separated chromatographically. Liebigs Ann. Chem. 521 (1987) and 1772 (1986) both isolate the desired components from Ginkgo tree leaves by means of adsorption on an ion exchange resin and desorption with acetone. Ginkgolides-Chemistry, Biology, Pharmacology and Clinical Perspectives, 1988, J. R. Prous Science Pub. S. A page 40 mentions column chromatography using chloroform for the isolation of pure ginkgolides.

However, any method of isolating ginkgolides from the leaves of the Ginkgo tree has not been found so far, because there is a small quantity of ginkgolides present in the leaves of the Ginkgo tree and it is difficult to isolate them.

Consequently, the purpose of the present invention is to provide a method of isolating the effective physio-active substances ginkgolides from the leaves of the Ginkgo tree and purifying them, at a high yield.

Detailed description of the present invention is as follows.

The present invention is a method of isolating ginkgolides from the leaves of the Ginkgo tree which is characterized by a series of processes as follows:
a) to adjust the pH of an aqueous solution of an extract of the leaves of the Gingko tree to the range of 1 to 9 by adding an alkaline aqueous solution,
b) to extract the aqueous solution treated as above with lower acetates, lower ketones or benzenes, separate the solution into a layer of aqueous solution and a layer of organic solvent, dehydrate and dry the layer of organic solvent, and
c) to adjust the pH of the layer of aqueous solution separated in the afore-said process b to the range of 1 to 3 by adding an acidic aqueous solution, extract the afore-said layer with lower ethers, chloroform or dichloromethane, dehydrate and dry the obtained extract,
d) to dissolve the dry powder obtained in afore-said process b and the residue obtained in the afore-said process c in a lower alcohol,
e) to add an aqueous solution of lead acetate to the solution obtained in the afore-said process d, remove the precipitate, concentrate the filtrate, and obtain a mixture powder of ginkgolides,
f) to purify the obtained mixture powder of ginkgolides
   I) either by passing the afore-said mixture powder through an adsorption column of activated charcoal and silicagel using a mixture solvent of lower ketones and lower alcohols and putting the obtained residue through separation recrystallization using anhydrous alcohol and an aqueous solution of acetone,
   II) or by dissolving the afore-said mixture powder in lower ketones or lower alcohols, adding distilled water to the obtained solution, centrifuging the solution thus treated and then putting the obtained residue through separation recrystallization using anhydrous alcohol and an aqueous solution of acetone.

In the present invention, the aqueous solution of an extract of the leaves of the Ginkgo tree is prepared by extracting a dry powder of the leaves of the Ginkgo tree in lower alcohols or lower ketones at the temperature of 50 to 100 °C for 5 to 6 h.

More detailed description of the present invention is as follows:

First, a powder of dried and pulverized fresh leaves of the Gingko tree is put in an aqueous solution of lower alcohols or lower ketones and then extracted at the temperature of 50 to 100 °C for 5 to 6 h. The filtrate thus obtained is mixed with an appropriate amount of carbon tetrachloride, which is water-insoluble, then agitated and then separated.

The pH of the layer of aqueous solution separated above is adjusted to the range of 1 to 9 by adding an aqueous solution of sodium hydroxide, then extracted with lower ketones, lower acetates or benzene, which are water-insoluble, then dehydrated by adding anhydrous sulfates, calcium carbonate or phosphates and then dried by depression.

The pH of the remaining layer of aqueous solution in the afore-said process is adjusted to the range of 1 to 3 by adding diluted hydrochloric acid and then extracted with a water-insoluble lower ether, chloroform or dichloromethane; the layer of organic solvent thus obtained is dried by depression and then mixed with the powder in the afore-said process.

The powder obtained in the afore-said process is dissolved in an aqueous solution of lower alcohols and then separated with hexane or cyclohexane to get rid of the remaining lipidic substances; to the filtrate thus obtained, an aqueous solution of lead acetate is added; the filtrate thus treated is agitated and let to stand; the precipitate thus formed is filtrated; by concentrating and drying the filtrate thus obtained, a mixture powder with a high content of ginkgolides is obtained.

Further, to purify the mixture powder obtained according to the present invention, the afore-said mixture powder is passed through an adsorption column of activated charcoal and silica gel using a solvent mixture of lower ketones and lower alcohols, thereby getting rid of the remaining phenolic substances; the filtrate passed through the adsorption column is dried by evaporation, then dissolved in a small amount of anhydrous ethanol and let to stand at low temperature; thus, a white amorphous solid is obtained.

Instead of said purifying procedure the afore-said mixture powder may be dissolved in an aque-ous solution of acetone, and then centrifuged; the powder thus obtained is dissolved in an alcohol, adding an aqueous solution of acetone and then let to stand at low temperature; thus, white crystalline ginkgolides are obtained.

The white solid obtained in the afore-said process is a mixture comprising pure Ginkgolide A, B and C and a very small amount of Ginkgolide M and J without phenolic substances; the respective ginkgolides can be isolated by separation recrystallization using a mixture solvent of acetone and water, and identified by thin-layer chromatography or liquid chromatography and hydrogen nuclear magnetic resonance spectrum.

To describe the present invention in further detail, examples are given as follows:

### Example 1

To 50 l of 10 % acetone aqueous solution, 10 kg of dried powder of the leaves of the Ginkgo tree is added. The mixture is extracted two times for 5 h at 80 °C with an inverse flow and then filtrated.

The filtrate is concentrated to 1 I and then extracted three times with 2 1 of carbon tetrachloride a time, thereby getting rid of the remaining chlorophylls. The pH of the remaining layer of aqueous solution is adjusted to 7.5 by adding 1 N aqueous solution of sodium hydroxide. The layer of aqueous solution treated as above is extracted five times with 3 1 of ethylacetate a time, then the ethylacetate extracts are dehydrated with sodium sulfate, then concentrated and dried.

The pH of the remaining layer of aqueous solution obtained after the afore-said process is adjusted to 3 by adding 1 N aqueous solution of hydrochloric acid. The layer of aqueous solution treated as above is extracted five times with 3 l of ethylether a time, then dehydrated and dried by depression. The powder thus obtained is mixed with the powder obtained by the afore-said process; thereby, 120 g of yellow solid power is obtained.

The afore-said yellow solid powder is dissolved in 150 ml of ethanol, then mixed with an aqueous solution of lead acetate and 15 g of activated charcoal, then agitated and filtrated. The filtrate is dried by drepression; thereby, 90 g of mixture powder with a high content of ginkgolides is obtained.

100 g of the solid substance thus obtained are put in 100 ml of acetone and methanol in the ratio of 15 : 1, and then let to pass through an adsorption column filled with 50 g of activated charcoal and 1 kg of silica gel, thereby getting rid of the remaining phenolic substances and impurities. The solution passed through the adsorption column is dried by evaporation; thereby, 18 g whitish powder is obtained.

The afore-said whitish powder is completely dissolved in 50 ml of anhydrous ethanol at 60 °C and then let to stand at a low temperature for 10 h; thereby, 10 g of white amorphous crystals, pure ginkgolides, are obtained.

Ginkgolides are identified by thin-layer chromatography [silica gel; cyclohexane ethylacetate (50 : 50) as solvent; acetic anhydride spray yielding, after heating, a faint orange fluorescence at 365 nm]. The afore-said ginkgolides are a mixture comprising Ginkgolides A, B and C and a very small amount of Ginkgolides J and M. Each ginkgolide can be isolated by separation recrystallization with 50 % aqueous solution of acetone.

### Example 2

To 50 l of ethylacetate, 10 kg of dried powder of the leaves of the Ginkgo tree is added. The mixture is extracted two times for 6 h at 80 °C with an inverse flow and then filtrated.

The filtrate is completely concentrated, then mixed with 5 1 of 20 % methanol, then agitated and let to stand at 50 °C, and then filtrated, thereby getting rid of insoluble substances. The filtrate is extracted two times with 2 l of cyclohexane a time, thereby getting rid of lipidic substances. The pH of the remaining layer of aqueous solution is adjusted to 7.5 by adding 1 N aqueous solution of sodium hydroxide. The layer of aqueous solution treated as above is extracted five times with 3 l of water a time and five times with 3 1 of saturated methylethylketone a time, then the ketone extracts are dehydrated with anhydrous sodium sulfate, then concentrated and dried.

The pH of the remaining layer of aqueous solution obtained after the afore-said process is adjusted to 2.5 by adding 1 N aqueous solution of hydrochloric acid. The layer of aqueous solution treated as above is extracted three times with 3 1 of chloroform a time, then dehydrated and dried. The solid powder thus obtained is mixed with the powder obtained by the afore-said process; thereby, 125 g of yellow solid powder is obtained.

The afore-said yellow solid powder is dissolved in 150 ml of ethanol, then mixed with an aque-ous solution of lead acetate, then agitated and let to stand, and filtrated, thereby getting rid of the formed precipitate. The filtrate is concentrated and dried; thereby, 60 g of mixture powder with a high content of ginkgolides is obtained.

100 g of the powder obtained as above are dissolved in 200 ml of acetone. To the solution thus obtained, 800 ml of distilled water is slowly added with agitation. The whitish colloidal liquid thus formed is centrifuged.

After centrifuging, the supernatant fluid is removed, and the remaining substances are dried by depression; thereby, 65 g of brown solid powder is obtained.

The afore-said solid powder is completely dissolved in 50 ml of 30 % methanol at 60 °C and then let to stand at a low temperature for 12 h; thereby, 17.5 g of white amorphous crystals, pure ginkgolides, are obtained.

The analysis by liquid chromatography (column: Waters µ - Bondapak C₁₈, length: 30 cm, diameter: 3.9 m, temperature: 25 ° C, solvent: mixture of water, methanol and tetrahydrofuran in the ratio of 15 : 20 : 10, flow rate: 1.5 ml/min, sensor: UV 220 nm) of the afore-said ginkgolides show that the ginkgolides are a mixture of 35 % Ginkgolide A, 45 % Ginkgolide B, 12 % Ginkgolide C and 3 % others.

By the separation recrystallization of the afore-said ginkgolides with anhydrous ethanol and 50 % aqueous solution of acetone, each pure ginkgolide is obtained.

### Example 3

To 50 l of methanol, 10 kg of dried powder of the leaves of the Ginkgo tree is added. The mixture is extracted two times for 6 h at 10 °C with an inverse flow and then filtrated.

The filtrate is concentrated to 5 l and then extracted two times with 2 l of carbon tetrachloride a time, thereby getting rid of the layer of organic solvent. The pH of the remaining layer of aqueous solution is adjusted to 8.5 by adding 1 N aqueous solution of sodium hydroxide. The layer of aqueous solution treated as above is separated five times with 3 l of benzene a time, and then the layer of benzene thus obtained is dehydrated with ammonium sulfate and dried. The pH of the remaining layer of aqueous solution is adjusted to 2.0 by adding 1 N aqueous solution of hydrochloric acid. The layer of aqueous solution treated as above is extracted three times with 2 l of dichloromethane a time, and the layer of organic solvent thus obtained is dehydrated and dried. The powder thus obtained is mixed with the powder obtained by the afore-said process; thereby, 70 g of brown powder is obtained.

The afore-said brown powder is dissolved in 120 ml of ethanol, then mixed with an aqueous solution of lead acetate, then agitated and let to stand, and filtrated, thereby getting rid of the formed precipitate. The filtrate is concentrated and dried; thereby, 55 g of mixture powder with a high content of ginkgolides is obtained.

100 g of the powder obtained as above are dissolved in 200 ml of acetone. To the solution thus obtained, 800 ml of distilled water is slowly added with agitation. The whitish colloidal liquid thus formed is centrifuged.

After centrifuging, the supernatant fluid is removed, and the remaining substances are dried by depression; thereby, 65 g of brown solid powder is obtained.

The afore-said solid powder is completely dissolved in 50 ml of 30 % methanol at 60 °C and then let to stand at a low temperature for 12 h; thereby, 17.5 g of white amorphous crystals, pure ginkgolides, are obtained.

The analysis by liquid chromatography (column: Waters µ - Bondapak C₁₈, length: 30 cm, diameter: 3.9 m, temperature: 25 °C, solvent: mixture of water, methanol and tetrahydrofuran in the ratio of 15 : 20 : 10, flow rate: 1.5 ml/min, sensor: UV 220 nm) of the afore-said ginkgolides show that the ginkgolides are a mixture of 35 % Ginkgolide A, 45 % Ginkgolide B, 12 % Ginkgolide C and 3 % others.

By the separation recrystallization of the afore-said ginkgolides with anhydrous ethanol and 50 % aqueous solution of acetone, each pure ginkgolide is obtained.

The results of analysis of each pure ginkgolide are as follows:

Ginkgolide A [melting point: 308 °C, Hydrogen Nuclear Magnetic Resonance Spectrum data (100 MHz, ppm): 1.20 (s), 1.45 (d), 2.0 - 2.5 (m), 2.60 (dd), 2.99 (dd), 3.44 (g), 5.05 (s), 5.13 (t), 5.38 (s), 6.20 (s), Infra-Red Adsorption Spectra data (*v* CO: 1802, 1116, 1164 cm⁻¹, *v* OH: 3551, 3453 cm⁻¹)],

Ginkgolide B [melting point: 300 °C, Hydrogen Nuclear Magnetic Resonance Spectrum data (100 MHz, ppm): 1.22 (s), 1.44 (d), 2 - 2.1 (m), 3.36 (g), 4.55 (d), 5.10 (d), 5.49 (s), 5.11 (s), 6.21 (s), Infra-Red Adsorption Spectra data (*v* CO: 1195, 1180 cm⁻¹, *v* OH: 3054 cm⁻¹)],

Ginkgolide C [melting point: 300 °C, Hydrogen Nuclear Magneteic Resonance Spectrum data (100 MHz, ppm): 1.32 (s), 1.45 (d), 2.14 (d), 3.36 (g), 4.53 (d), 4.13 (dd), 5.05 (d), 5.53 (s), 5.61 (d), 6.30 (s), Infra-Red Adsorption Spectra data (*v* CO: 1185, 1163 cm⁻¹, *v* OH: 3519, 3529 cm⁻¹)].

## Claims

1. A method of isolating ginkgolides from the leaves of the Ginkgo tree which is **characterized by** a series of steps as follows:
a) to adjust the pH of an aqueous solution of an extract of the leaves of the Ginkgo tree to the range of 1 to 9 by adding an alkaline aqueous solution,
b) to extract the aqueous solution treated as above with lower acetates, lower ketones or benzenes, separate the solution into a layer of aqueous solution and a layer of organic solvent, dehydrate and dry the layer of organic solvent, and obtain a powder,
c) to adjust the pH of the layer of aqueous solution separated in the afore-said process b to the range of 1 to 3 by adding an acidic aqueous solution, extract the layer of aqueous solution treated as above with lower ethers, chloroform or dichloromethane, dehydrate and dry the obtained extract,
d) to dissolve the dry powder obtained the afore-said process b and the residue obtained in the afore-said process c in a lower alcohol,
e) to add an anqueous solution of lead acetate to the solution obtained in the afore-said process d, remove the precipitate, concentrate the filtrate, and obtain a mixture powder of ginkgolides,
f) to purify the obtained mixture powder of ginkgolides
I) either by passing the afore-said mixture powder through an adsorption column of activated charcoal and silicagel using a mixture solvent of lower ketones and lower alcohols and putting the obtained residue through separation recrystallization using anhydrous alcohol and an aqueous solution of acetone,
II) or by dissolving the afore-said mixture powder in lower ketones or lower alcohols, adding distilled water to the obtained solution, centrifuging the solution thus treated and then putting the obtained residue through separation recrystallization using anhydrous alcohol and an aqueous solution of acetone.

2. A method according to Claim 1 wherein the aqueous solution of an extract of the leaves of the Ginkgo tree is prepared by extracting a dry powder of the leaves of the Ginkgo tree in lower alcohols or lower ketones at the temperature of 50 to 100 °C for 5 to 6 h.

3. A method according to Claim 1 wherein the layer of organic solvent in the afore-said process b is dehydrated and dried by adding anhydrous sulfates, calcium carbonate or phosphates.

## Patentansprüche

1. Verfahren zur Gewinnung von Ginkgoliden aus den Blättern des Ginkgo-Baumes, **gekennzeichnet durch** eine Reihe von Stufen, in denen man
a) den pH-Wert einer wäßrigen Lösung eines Extraktes der Blätter des Ginkgo-Baumes auf den Bereich von 1 bis 9 einstellt, indem man eine alkalische wäßrige Lösung zugibt,
b) die wäßrige, wie oben behandelte Lösung mit niedermolekularen Acetaten, niedermolekularen Ketonen oder Benzolen extrahiert, die Lösung in eine wäßrige Lösungsschicht und eine organische Lösungsschicht trennt, die organische Lösungsschicht entwässert und trocknet und
c) den pH-Wert der wäßrigen Lösungsschicht, die in dem obengenannten Verfahren b abgetrennt wurde, auf den Bereich von 1 bis 3 einstellt, indem man eine saure wäßrige Lösung zugibt, die obengenannte Schicht mit niedermolekularen Ethern, Chloroform oder Dichlormethan extrahiert, den erhaltenen Extrakt entwässsert und trocknet,
d) das in dem obenerwähnten Verfahren b erhaltene trockene Pulver und den in dem obenerwähnten Verfahren c erhaltenen Rückstand in einem niedermolekularen Alkohol löst,
e) eine wäßrige Lösung von Bleiacetat zu der in dem obengenannten Verfahren d erhaltenen Lösung zusetzt, den Niederschlag entfernt, das Filtrat konzentriert und ein gemischtes Pulver von Ginkgoliden erhält,
f) das erhaltene Pulvergemisch von Ginkgoliden reinigt, indem man
l) entweder das obengenannte Pulvergemisch durch eine Adsorptionssäule von aktivierter Kohle und Kieselgel unter Verwendung eines Lösungsmittelgemisches von niedermolekularen Ketonen und niedermolekularen Alkoholen schickt und den erhaltenen Rückstand durch Umkristallisation unter Verwendung eines wasserfreien Alkohols und einer wäßrigen Acetonlösung trennt,
II) oder indem man das obenerwähnte Pulvergemisch in niedermolekularen Ketonen oder niedermolekularen Alkoholen auflöst, destilliertes Wasser zu der erhaltenen Lösung zugibt, die so behandelte Lösung zentrifugiert und dann den erhaltenen Rückstand durch Umkristallisieren unter Verwendung von wasserfreiem Alkohol und einer wäßrigen Acetonlösung trennt.

2. Verfahren nach Anspruch 1, bei dem die wäßrige Lösung eines Extraktes der Blätter des Ginkgo-Baumes durch Extrahieren eines Trockenpulvers der Blätter des Ginkgo-Baumes in niedermolekularen Alkoholen oder niedermolekularen Ketonen bei einer Temperatur von 50 bis 100 °C während 5 bis 6 h hergestellt wird.

3. Verfahren nach Anspruch 1, bei dem die organische Lösungsmittelschicht in dem Verfahren b durch Zugabe von wasserfreien Sulfaten, Calciumcarbonat oder Phosphaten entwässert und getrocknet wird.

## Revendications

1. Procédé pour isoler des ginkgolides à partir des feuilles de ginkgo, qui est caractérisé par une série d'étapes comme suit:
a) ajuster le pH d'une solution aqueuse d'un extrait de feuilles de ginkgo dans le domaine de pH de 1 à 9 par addition d'une solution aqueuse alcaline,
b) extraire la solution aqueuse traitée comme indiqué ci-dessus avec des acétates inférieurs, des cétones inférieures ou des benzènes, séparer la solution en une phase de solution aqueuse et une phase de solvant organique, déshydrater et sécher la phase de solvant organique, et obtenir une poudre,
c) ajuster le pH de la phase de solution aqueuse isolée dans le processus b précité dans le domaine de pH de 1 à 3 en ajoutant une solution aqueuse acide, extraire la phase de solution aqueuse traitée comme décrit ci-dessus avec des éthers inférieurs, du chloroforme ou du dichlorométhane, déshydrater et sécher l'extrait obtenu,
d) dissoudre la poudre sèche obtenue dans le processus b précité et le résidu obtenu dans le processus c précité dans un alcool inférieur,
e) ajouter une solution aqueuse d'acétate de plomb à la solution obtenue dans le processus d précité, éliminer le précipité, concentrer le filtrat, et obtenir une poudre de mélange de ginkgolides,
f) purifier la poudre de mélange de ginkgolides obtenue
I) soit en faisant passer la poudre de mélange précitée à travers une colonne d'adsorption constituée de charbon actif et de silicagel utilisant un mélange de solvants constitué de cétones inférieures et d'alcools inférieurs et en soumettant le résidu obtenu à l'étape de recristallisation par séparation à l'aide d'un alcool anhydre et d'une solution aqueuse d'acétone,
II) soit en dissolvant la poudre de mélange précitée dans des cétones inférieures ou des alcools inférieurs, en ajoutant de l'eau distillée à la solution obtenue, en centrifugeant la solution ainsi traitée et ensuite en soumettant le résidu obtenu à l'étape de recristallisation par séparation à l'aide d'alcool anhydre et d'une solution aqueuse d'acétone.

2. Procédé selon la revendication 1, dans lequel la solution aqueuse d'un extrait de feuilles de ginkgo est préparée par extraction d'une poudre sèche de feuilles de ginkgo dans des alcools inférieurs ou des cétones inférieures, à une température de 50 et 100°C pendant 5 à 6 h.

3. Procédé selon la revendication 1, dans lequel la phase de solvant organique dans le processus b précité est déshydratée et séchée par addition de sulfates anhydres, de carbonate de calcium ou de phosphates.
